Europäisches Patentamt

European Patent Office

Office européen des brevets

(11) EP 1 459 718 A1

(12) EUROPEAN PATENT APPLICATION

(43) Date of publication:
22.09.2004 Bulletin 2004/39

(51) Int Cl.7: A61F 13/15

(21) Application number: 04006386.9

(22) Date of filing: 17.03.2004

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL LT LV MK**<br><br>(30) Priority: **20.03.2003 JP 2003076677**<br><br>(71) Applicant: **KAO CORPORATION**<br>**Chuo-ku, Tokyo (JP)**<br><br>(72) Inventors:<br>• **Itoh, Taketo, c/o Kao Corp., Res. Lab.**<br>**Tochigi (JP)** | • **Kasai, Takao, c/o Kao Corp., Res. Lab.**<br>**Tochigi (JP)**<br>• **Okuda, Yasuyuki, c/o Kao Corp., Res. Lab.**<br>**Tochigi (JP)**<br>• **Toyoshima, Haruko, c/o Kao Corp., Res. Lab.**<br>**Tochigi (JP)**<br><br>(74) Representative: **VOSSIUS & PARTNER**<br>**Siebertstrasse 4**<br>**81675 München (DE)** |

(54) **Disposable diaper**

(57) A flat type disposable diaper with a pair of waistbands, which has a pair of standing gathers formed on longer sides thereof and contains a region having a lateral bending stiffness of 25 cN/50 mm or less within a region having an absorbent member in the crotch portion. The standing gathers are fixed at an extension ratio of 100% or higher and have such tensile characteristics in their state not fixed to the diaper that the tensile load required to extend to an effective extension ratio is 20 to 120 cN and that the increase rate of tensile load required for extending from an extension ratio of 20% up to the effective extension ratio is 1.0 cN/% or lower, the effective extension ratio being 30% lower than the fixing extension ratio.

Fig.1

Printed by Jouve, 75001 PARIS (FR)

**Description**

The present invention relates to a disposable diaper.

Disposable diapers of flat type composed of a liquid permeable topsheet, a liquid impermeable backsheet, and a liquid retentive absorbent member interposed between the two sheets are widely used. The disposable diapers of this type have a front portion that is to be applied to the stomach of a wearer and a rear portion that is to be applied to the wearer's back.

Among the known flat type disposable diapers are those designed for enabling an adult wearer to put on while in a standing posture, which have waistband portions oppositely disposed on the sides of the rear portion (see JP-A-5-184623 and JP-A-9-290002). At least one of the waistband portions has a band fastening member near the free end thereof, and the front portion has a front fastening member on both sides thereof. A wearer or one placing the diaper first fastens the opposite band portions together on the wearer's stomach side with the band fastening member and then fastens the front portion to the waistband portions with the front fastening members.

The conventional diaper design using the waistband portions makes it possible for a wearer or one placing the diaper to put the diaper on while the wearer is standing. However, when a wearer puts on a diaper by himself or herself, the conventional disposable diaper is not so easy to put on. In particular, it is not easy for a wearer to bring the front portion to the wearer's front after fastening the waistband portions together. In order to improve ease of putting on, the portion to be located at the wearer's crotch (crotch portion) could be narrowed, but it is difficult to narrow the crotch portion without inviting reduction in absorbing capability and leakproofness that are essentially required of diapers.

If narrowing the crotch portion is adopted to a so-called fitted disposable diaper having a pair of standing gathers (or cuffs) and a pair of leg gathers oppositely disposed on the longer sides of the diaper, the distance between the opposite standing gathers must be reduced, which results in insufficient absorbing performance. If the crotch portion is narrowed without reducing the distance between the standing gathers, there would no pocket left between the standing gathers and the respective leg gathers, and body waste leakage can occur easily.

The disposable diaper of the present invention is a flat type diaper having a rear portion that is to be applied to the back of a wearer, a front portion that is to be applied to the stomach of the wearer, and a crotch portion located between the rear and front portions. The rear portion has a waistband portion oppositely extending from each side thereof. At least one of the waistband portions has a band fastening member near the free end thereof, with which the opposite waistbands are fastened together on the front side of a wearer.

The disposable diaper according to a first aspect of the invention has a pair of standing gathers formed on the longer sides of the diaper and contains a region having a lateral (in the diaper width direction) bending stiffness of 25 cN/50 mm or lower in a region having the absorbent member in the thickness direction in the crotch portion.

The standing gathers are formed by fixing at an extension ratio (hereinafter defined) of 100% or higher. The extension ratio in fixing the standing gathers will hereinafter be sometimes referred to as a fixing extension ratio. The tensile characteristics of the standing gathers on each side measured in their state not fixed to the diaper are such that the tensile load required to extend to an effective extension ratio that is 30% lower than the fixing extension ratio is 20 to 120 cN and that the increase rate of tensile load required for extending from an extension ratio of 20% up to the effective extension ratio is 1.0 cN/% or lower.

The disposable diaper according to a second aspect of the invention is also a flat type and waistband type diaper. This disposable diaper has a pair of standing gathers and a pair of leg gathers oppositely formed by fixing respective elastic members on the longer sides thereof. The smallest width of the crotch portion is 100 to 240 mm. The ratio of the distance W1 between the opposite fixed ends of the pair of standing gathers to the distance W2 between the opposite elastic members that are arranged most outwardly in the leg gathers, W1/W2, both measured at the smallest width of the crotch portion is 0.67 to 0.81. The distance W3 between the fixed end of the standing gathers on each side of the diaper and the elastic member that is arranged most outwardly in the leg gathers on the same side of the diaper as measured at the smallest width of the crotch portion is smaller than the width W4 of the standing gathers on each side as measured at the smallest width of the crotch portion.

In the diaper according to the second aspect of the invention, the standing gathers are fixed at an extension ratio of 100% or higher, and the tensile characteristics of the standing gathers on each side measured in their state not fixed to the diaper are such that the tensile load required to extend to an effective extension ratio that is 30% lower than the fixing extension ratio is 20 to 120 cN and that the increase rate of tensile load required for extending from an extension ratio of 20% up to the effective extension ratio is 1.0 cN/% or lower.

The present invention will be more particularly described with reference to the accompanying drawings, in which:

Fig. 1 is a plan view of a disposable diaper according to an embodiment of the present invention (including the first and second aspects), with every elastic member stretched flat.

Fig. 2 is a schematic cross-sectional view of the disposable diaper of Fig. 1, taken along line X-X of Fig. 1 where the crotch portion is narrowest.

Fig. 3 is a schematic plan view of an embodiment of the present invention showing stiffness design of the region where an absorbent member lies in the crotch portion.

Fig. 4 shows the disposable diaper of Fig. 1 being put on a wearer in the state after the waistband portions have been fastened together on the front side of the wearer.

Fig. 5 shows the disposable diaper of Fig. 1 having been put on a wearer.

Figs. 6 (a) through 6(d) show other stiffness designs applicable to a disposable diaper according to embodiments of the present invention.

Fig. 7 is a graph showing the relationship between extension ratio and tensile stress (load) in standing gathers of a disposable diaper in their non-fixed state.

The present invention relates to a flat type disposable diaper having waistband portions on each side thereof which, when the diaper is put on a wearer, are fastened together on the wearer's front or back side. The disposable diaper of the present invention is easy to put on a wearer either in a lying or a standing posture and excellent in absorbing performance and leakproofness.

The present invention will be illustrated in greater detail based on its preferred embodiments with reference to the accompanying drawings.

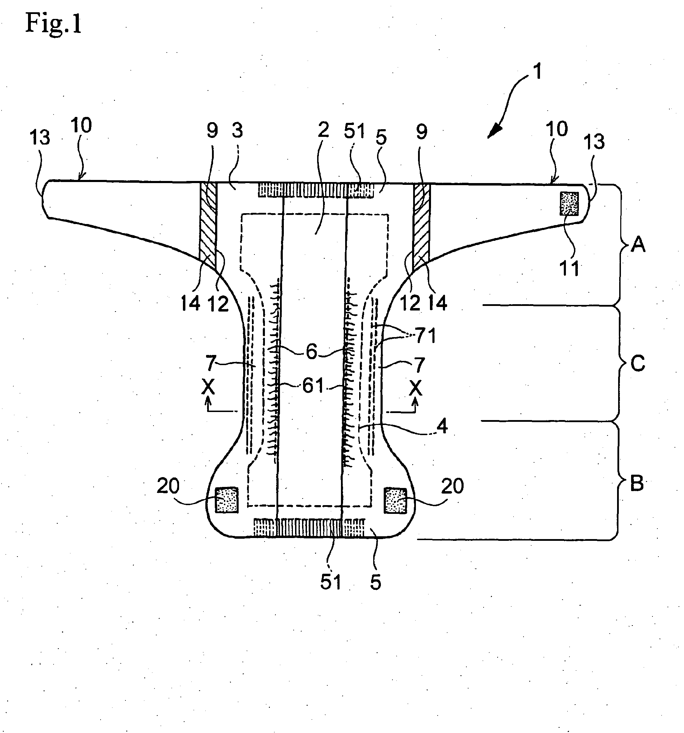

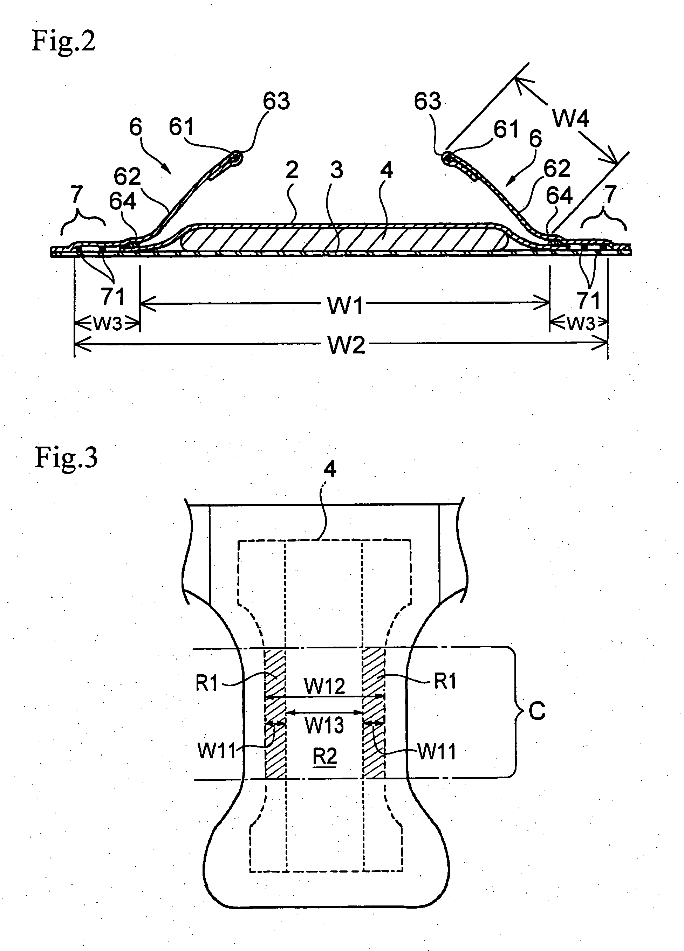

Figs. 1 and 2 show an embodiment of the disposable diapers according to the first and second aspects of the invention. The disposable diaper 1 of this embodiment is a waistband type that can be put on a standing wearer. As shown in Figs. 1 and 2, the disposable diaper 1 is substantially longer than wide and has a liquid permeable topsheet 2, a liquid impermeable backsheet 3, and a liquid retentive absorbent member 4 disposed between the sheets 2 and 3. The disposable diaper 1 is sectioned into a rear portion A that is to be applied to the back of a wearer, a front portion B that is to be applied to the stomach of the wearer, and a crotch portion C located between the rear portion A and the front portion B. The crotch portion C is to be applied to the crotch of a wearer and corresponds to the middle section when the length of the diaper is divided into substantially equal thirds.

The absorbent member 4 is fixedly disposed between the topsheet 2 and the backsheet 3. A waist elastic member 51 for ensuring a snug fit to a wearer is provided on the waist portion 5 which is located outside the longitudinal ends of the absorbent member 4.

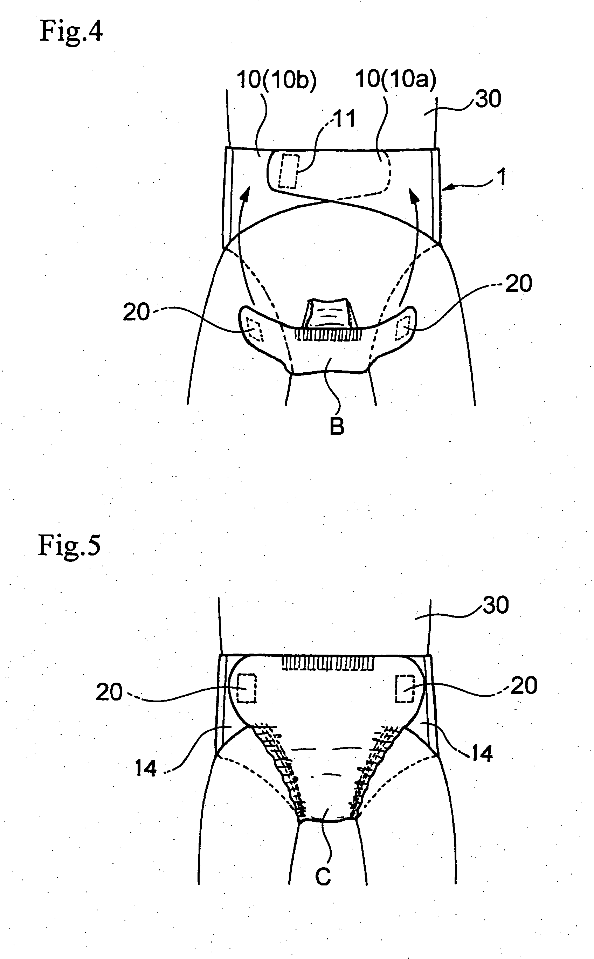

The rear portion A has lateral sides 9, and a waistband portion 10 is connected to each of the sides 9. At least one of the waistband portions 10 has a band fastening member 11 near its free end 13 so that the opposite waistband portions 10 (10a and 10b as shown in Fig. 4) may be fastened together on the wearer's front as illustrated in Figs. 4 and 5.

A pair of front portion fastening members 20 are provided on the lateral sides of the topsheet 2 in the front portion B, whereby the front portion B can be joined to the waistband portions 10 fastened together as illustrated in Fig. 4. The width of each waistband portion 10 gradually decreases from the fixed end 12 toward the free end 13. Each waistband portion 10 includes an elastically stretchable part 14 in at least the side of the fixed end 12. The elastically stretchable part 14 is formed of a laminate sheet composed of an elastic sheet made of an elastomeric

material and an extensible fiber aggregate integrally overlaid on one or both sides of the elastic sheet such that the extensible fiber aggregate may extend in the diaper width direction. The whole length of the waistband portion may be formed of such an elastically stretchable laminate sheet. The above-described structure can be seen in conventional disposable diapers, and each of the members constituting the structure can be made of known materials with no particular restriction.

The band fastening member 11 and the front portion fastening members 20 are preferably formed of a male member of a mechanical fastener having a large number of anchor-shaped or J-shaped hooks on the surface thereof. Commercially available male members of mechanical fasteners can be used, including Magic Tape® (from Kuraray Co., Ltd.), Quicklon® (from YKK Co., Ltd.), and Magicloth® (from Kanebo BELL-TOUCH, Ltd.). At least the backsheet side of the waistband portions 10 is made of nonwoven fabric so that the male member of the mechanical fastener may be directly engaged thereon.

The disposable diaper 1 has a pair of standing gathers 6 and a pair of leg gathers 7 oppositely formed on both longer sides of the diaper by disposing the respective elastic members. Specifically, as shown in Fig. 2, the standing gathers 6 are formed of respective gather-forming sheets 62 having respective elastic members 61, the gather-forming sheet 62 being disposed to cover both longer side areas of the topsheet 2. Each sheet 62 is linearly fixed at one longer side thereof to the topsheet 2 along the diaper length direction by known bonding means such as heat seal or an adhesive. The linear joint of the sheet 62 with the topsheet 2 forms a fixed end 64 of the standing gathers 6. The part of the sheet 62 extending outward from the fixed end 64 is fixed to the topsheet 2 or the backsheet 3. In the areas near the two longitudinal ends of the diaper 1, the part of the sheet 62 extending inward from the fixed end 64 is bonded to the topsheet 2. The elastic member 61 used to form the standing gathers 6 is an elastic thread, which is arranged substantially in parallel with the free end 63 of the standing gathers 6.

The pair of leg gathers 7 are formed by disposing a plurality of leg elastic members 71 in an almost linear pattern outward the fixed end 64 of the respective standing gathers 6. Each of the elastic members 61 and 71 is parallel to the diaper length.

As shown in Fig. 3, the diaper 1 according to the present embodiment has regions R1 having a lateral bending stiffness of 25 cN/50 mm or lower in the region having the absorbent member 4 in the crotch portion C. More specifically, in the crotch portion C, the region where the absorbent member 4 is disposed (hereinafter referred to as a region R) contains regions R1 indicated by slant lines in Fig. 3 which are less stiff (softer) than the other region R2 in the region R. The region R1 will hereinafter be called a low stiffness region R1. The low stiffness regions R1 have a lateral (in the diaper width direction) bending stiffness of 25 cN/50 mm or lower. The term "region" as in "the region having the absorbent member 4 in the crotch portion C (region R)" or as in "the low stiffness region" as used herein means a three-dimensional part that contains the absorbent member 4 in its thickness (between the upper and lower surfaces of the diaper) when stretched flat and seen from above as in Fig. 1.

In the embodiment shown in Fig. 3, each of the low stiffness regions R1 is an oblong region provided along each longer side edge of the absorbent member 4 in the region R.

The region R in the crotch portion C is required to have sufficient width and space for collecting and retaining body waste within the crotch portion while worn and should have a minimum restoring force for maintaining such width and space. In addition, when an adult wearer puts on the disposable diaper by him or herself, it is desirable that the front portion be easily drawn up between the legs toward the front side and pulled upward in the front after the waistband portions are fastened together. All these taken into consideration, the lower limit of bending stiffness of the low stiffness regions R1 is preferably about 3 cN/50 mm, more preferably about 5 cN/mm. For assuring ease of diapering without sacrificing the fit and safety against leakage, a preferred bending stiffness of the low stiffness region R1 is 3 to 25 cN/50 mm, particularly 5 to 20 cN/50 mm. By this stiffness design, the front portion B can be fastened to a target position more easily, and the diaper can be put on in the right wearing position without causing the first fastened waistband portions to slide down.

In the diaper 1 of the present embodiment, the region R also has a region R2 having a width W13 (see Fig. 3) of at least 50 mm across the diaper and a lateral bending stiffness of higher than 25 cN/50 mm (hereinafter referred to as a high stiffness region R2). From the standpoint of ease of diapering and shape retention in use, a preferred upper limit of the bending stiffness of the high stiffness region R2 is 60 cN/50 mm, and a more preferred bending stiffness of the high stiffness region R2 is 30 to 50 cN/50 mm.

To improve ease for a wearer to put on the diaper in a standing posture or to place a diaper on a wearer in a standing position, the width W11 (see Fig. 3) of each low stiffness region R1 preferably ranges 15 to 50% of the width W12 (see Fig .3) of the region R, and the total width of the low stiffness regions R1 preferably ranges 30 to 100% of the width W12. The width W13 (see Fig. 3) of the high stiffness region R2 between the pair of the low stiffness regions R1 is preferably 0 to 70% of the width W12. The width W12 is preferably 70 mm or larger in order to secure sufficient absorbency of the crotch portion. The region R width of 70 mm or larger is also helpful for a wearer to bring the front portion B to the wearer's front between his or her legs and pull it up to a target position after fastening the opposite waistband portions together on the stomach side. From the standpoint of ease of diapering and fit to the wearer's crotch, the width W12 is preferably 150 mm or smaller. The smaller the width W13, the more preferred. The width W13 is preferably 0 to 80 mm.

The lateral (in the diaper width direction) bending stiffness of the low stiffness region R1 and the high stiffness region R2 is measured as follows.

Measurement of bending stiffness:

Standing gathers are removed from a diaper. A 50 mm wide and 80 mm long rectangular specimen containing all the constituent members including from the topsheet to the backsheet in its thickness direction is cut out of the region R. The width (50 mm) and the length (80 mm) of the specimen are in the length and the width directions of the diaper, respectively. Care should be taken so that the specimen may have no crease or wrinkle that may influence the measurement.

Measurement is carried out with a Tensilon tester, RTC-1150A supplied by Orientec, equipped with a 5-kg load cell (range: 200 cN) in accordance with JIS (Japanese Industrial Standards) K7171 (Plastics—Determination of flexural properties) (R1=5.0±0.1 mm, R2=±0.2 mm). The specimen is placed on two supports 50 mm apart, with its length being parallel to the line connecting the two supports, and an indenter is positioned to just touch the mid-point of the specimen. The indenter is moved down at a speed of 30 mm/min to obtain a load-deformation curve. The maximum of the bending stress is taken as a bending stiffness (cN/50 mm). The "50 mm" in the unit of the bending stiffness corresponds to the width of the specimen bent by the indenter.

Where the region R1 or R2 to be measured is greater than 50 mm across the diaper, a specimen is cut out such that the whole width of the region is contained in the specimen length. Where the region R1 or R2 is equal to or shorter than 50 mm across the diaper, a specimen is cut out so as to contain the whole width of the region in the specimen length and is placed over the supports with at least part of that region positioned between the support. For instance, in measuring the bending stiffness of a low stiffness region R1 arranged on each side of the region R, where the width of the low stiffness region R1 is 50 mm or smaller, a specimen is cut out such that the low stiffness region R1 is present in one longitudinal end portion of the specimen and placed on the supports such that one end of the low stiffness region R1 is put on one of the supports and the other end between the supports. In this case, the mid-point of the specimen, at which the indenter presses, may be outside of the low stiffness region R1. Nevertheless the measured maximum load is regarded as the bending stiffness (cN/50 mm) of the low stiffness region R1 because the maximum load is predominantly governed by the stiffness of the low stiffness region R1.

Where a specimen containing a region whose width exceeds 50 mm in the diaper width direction shows a maximum load exceeding 25 cN/50 mm as measured in the same manner as described above, the region R has a high stiffness region R2 having a width 13 (see Fig. 3) of 50 mm or larger and a lateral bending stiffness exceeding 25 cN/50 mm.

The low stiffness region R1 in the present embodiment is formed by varying the constitution of part of the absorbent member 4 within the region R from the constitution of the other part in the region R, more specifically, by reducing the basis weight of part of the absorbent member 4 than that of the other part.

Methods of reducing the basis weight of part of an absorbent member than that of the other part include the following. (1) An absorbent member is fabricated of an upper absorbent layer and a lower absorbent layer, and part of the absorbent member is formed solely of either the upper or the lower absorbent layer to provide a low stiffness region. (2) An absorbent member is fabricated of fibers air-laid by suction on a net, and the amount of the fibers to be air-laid is made smaller in part than in the other part. (3) An absorbent member is fabricated of three or more absorbent layers, and part of the absorbent member has a smaller number of absorbent layers by at least one layer than the other part. (4) An absorbent member is fabricated of two or more absorbent layers, and part of the absorbent member lacks one or more constituents. Preferred of them is the method (1) because the upper and the lower absorbent layers can be prepared separately, which makes it relatively easy to control the absorbing performance and the basis weight as designed.

The standing gathers 6 are formed by fixing a low modulus material at a high extension ratio. The term "fixing extension ratio" as used herein means an extension ratio at which a standing gathers-forming sheet having an elastic member is fixed to the main body of a diaper. In the present embodiment, the standing gathers 6 are fixed in a stretched state at a fixing extension ratio of 100% or higher, preferably 100 to 300%, more preferably 130 to 200%.

If the fixing extension ratio of the standing gathers is less than 100%, the resultant gathers do not rise up sufficiently in a fitted diaper. It follows that the standing gathers 6 may fall or bend when the free end of the standing gathers 6 is held to the wearer's crotch particularly in diapering a standing wearer, making correct diapering difficult. Besides, when a wearer changes his or her body position, there may be formed a gap between the free end of the gathers and the skin, through which body waste can leak.

If the fixing extension ratio is more than 300%, the disposable diaper can curl up excessively and be difficult to put on a wearer particular when the wearer is lying on its back. When changing after being soiled, the diaper would be difficult to handle due to its strong tendency to curl up. When such a curling diaper is put on a standing wearer, the curling makes fastening difficult.

The extension ratio of the standing gathers in fixing is measured as follows.

Measurement of fixing extension ratio:

A strip of the gathered sheet 62 having an elastic member (a portion between the fixed end and the free end) is cut off along the diaper length direction so that the strip (specimen) may contain at least the lengthwise middle of the gathers. The specimen is stretched to the maximum, i.e., to the stretched state as shown in Fig. 1. A distance between arbitrarily chosen two points, preferably about 200 mm apart, of the stretched specimen is taken as a stretched length H. The specimen is then put into the natural state, i.e., let to contract spontaneously, and a distance between the two points (natural length h) is measured. The fixing extension ratio is represented by the equation:

$$\text{Fixing extension ratio (\%)} = [(H-h)/h] \times 100$$

The natural length h is measured by placing the gathered strip on a flat surface while minimizing the unevenness due to gathering with minimal load applied thereto.

The tensile characteristics of the standing gathers 6 (either one of the pair) are such that the tensile load required to extend the standing gathers 6 in its non-fixed state (the state not fixed to the diaper, that is, the state after being cut off the diaper) to an effective extension ratio is 20 to 120 cN, preferably 50 to 100 cN, and that the increase rate of tensile load required for extending in the non-fixed state from an extension ratio of 20% up to the effective extension ratio is 1.0 cN/% or lower, preferably 0.7 cN/% or lower. The effective extension ratio is defined to be an extension ratio 30% lower than the fixing extension ratio.

The reason why the tensile characteristics of the standing gathers are evaluated at the effective extension ratio, (fixing extension ratio-30%), is that the extension (%) vs. load curve often contains the tensile load component attributed to the constituent members of the standing gathers in the region above the effective extension ratio. The part of the curve above the effective extension ratio is likely to represent a tensile stress in excess of the physical properties of the gathers *per se,* that is, false data. In addition, a fitted diaper with standing gathers is curved into a U-shape when put on, and the free end of the standing gathers disposed on the inner side of the diaper depicts a U-shape having a smaller curvature radius than the outer surface of the U-shaped diaper. Since the standing gathers are rarely stretched to the fixing extension ratio in actual use, it can be seen as reasonable to discuss the behavior of the standing gathers at a given extension ratio lower than the fixing extension ratio in evaluating the behavior in actual use.

The tensile characteristics (tensile load at the effective extension ratio and tensile load increase rate) of the standing gathers in a non-fixed state are measured as follows.

Measurement of tensile characteristics:

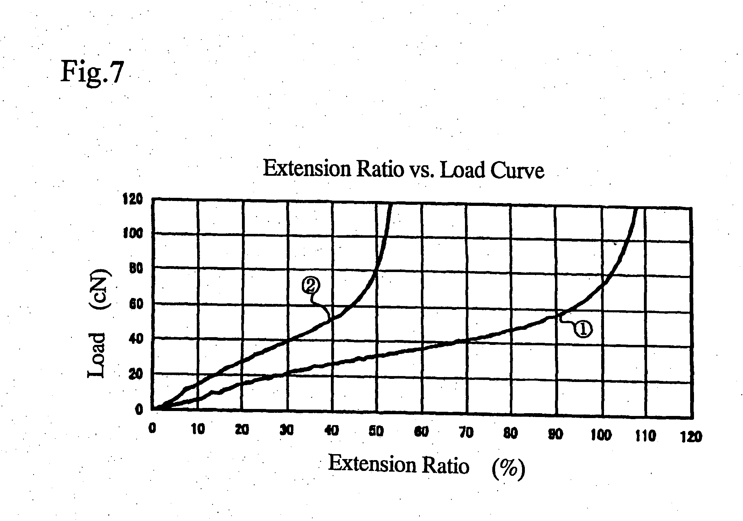

A strip of the gathered sheet 62 having an elastic member (a portion between the fixed end and the free end) is cut off its fixed end 64 such that the strip contains the lengthwise middle of the standing gathers 6 to prepare a specimen. The specimen should have a natural length of at least 70 mm as measured on a flat surface while minimizing the unevenness due to gathering with minimal load applied thereto. It is desirable to use the specimen used in the measurement of the fixing extension ratio. The specimen is clamped in the jaws of a Tensilon tensile tester, RTC-1150A supplied by Orientec, equipped with a 5 kg-load cell. The jaws are set at an initial distance of 50 mm, which is an initial natural length of the specimen at 0% extension. The specimen is extended up to the fixing extension ratio at a rate of 300 mm/min, and the extension ratios vs. increasing tensile load are plotted to obtain a curve with the extension ratio (%) as abscissa and the tensile load (cN) as ordinate (see Fig. 7).

The tensile loads at an extension ratio of 20% and an effective extension ratio are read from the curve. The slope of the curve from the extension ratio of 20% to the effective extension ratio, represented by (tensile load at effective extension ratio - tensile load at 20% extension ratio)/(effective extension ratio (%) - 20%), is calculated to obtain a tensile load increase rate (cN/%) of from 20% extension ratio to effective extension ratio.

If the tensile load at the effective extension ratio is less than 20 cN, the gathers do not rise up sufficiently in a fitted diaper. It follows that the standing gathers may fall or bend when the free end of the standing gathers is held to the wearer's crotch particularly when placing a diaper in a standing position, making correct diapering difficult. Besides, when a wearer changes his or her body position, a gap may be formed between the free end of the gathers and the skin, through which body waste can leak. Even when the free end of the pair of standing gathers is in close contact with the wearer's body, the squeezing force of the standing gathers is so weak that the force of confining discharged body waste in the space between the pair of standing gathers would be insufficient. It is likely to follow that the body waste leaks over the free end of the standing gathers. Therefore, the standing gathers with such a low tensile load may not be effective against leakage.

If the tensile load at the effective extension ratio is more than 120 cN, a large force is necessary to force open wide the U-shaped diaper and impairs the ease of placing a diaper on a wearer lying on his or her back. When changing after being soiled, the diaper is difficult to handle due to its strong tendency to curl up. Furthermore, in placing a diaper on a wearer in a standing position, the diaper may roll up before the waistband portions 10 are fastened together, making diapering difficult.

These problems occur when the elastic member is fixed at a high extension ratio as with the case of embodiments of the present invention. In other words, the inconvenience arises from the phenomenon that the standing gathers stretched while diapering exhibit contractibility to be relieved from the tensile stress and make the whole diaper roll-up or curl-up. Against the problems, the present invention has succeeded in assuring ease of diapering by reducing the tensile load to some extent thereby to suppress the behavior to contract, retard the contraction, or reduce the force necessary to open the contracted gathers wide. With the fixing extension ratio being equal, the tensile stress is a governing factor. The smaller the tensile stress, the more extensible the standing gathers and the easier to handle the diaper. Unless the tensile load at the effective extension ratio exceeds 120 cN, the manageability of the diaper is not impaired, and a wearer or a person handling the diaper is able to open the diaper wide with ease.

The tensile load increase rate is desirably as small as possible. With a smaller increase rate, the force of the standing gathers to come into intimate contact with the skin and to block body waste will be less changeable depending on the wearer's position or the way of diapering. Further, the diaper will be easier to handle, giving a wearer or a person handling the diaper no feeling of abrupt change in force necessary to open the diaper wide nor a heavy feeling in opening the diaper wide. As a result, the diaper will have improved ease in putting on whether a wearer is in a lying position or a standing position.

Accordingly, it is desirable that the tensile load increase rate, calculated as an increase in tensile load per unit increase in extension ratio, be 1.0 cN/% or lower at every point between 0% extension ratio up to an effective extension ratio. The tensile load increase rate may exceed 1.0 cN/% in some regions between 0% extension ratio up to an effective extension ratio. A diaper showing such a tensile load increase rate as exceeding 1.0 cN/% is not deemed to be a departure from the scope of the present invention as long as such a high increase rate is observed in the region of from 0% extension ratio to 20% extension ratio. Nevertheless, the upper limit of the tensile load increase rate in the region from 0 to 20% extension ratio is preferably 2.0 cN/%.

The above is the reason the tensile load increase rate in the region from 20% extension to an effective extension ratio is specified in the present invention.

For application to infants, the smallest width of the diaper 1 in the crotch portion is preferably 100 to 240 mm. As long as the smallest width is 100 mm or greater, sufficient width and space for collecting and retaining body waste are secured in the crotch portion. When the smallest width is less than 240 mm, the front portion B can easily be brought to the wearer's front and pulled up to be fastened after the waistband portions are fastened together when the diaper is placed on a wearer in a standing position, particularly by the wearer himself or herself. For ease of placing a diaper on a wearer in a standing position, the smallest width of the crotch portion C is more preferably 100 to 230 mm, even more preferably 100 to 210 mm, even more preferably 120 to 200 mm.

For application to adults, on the other hand, the smallest width of the crotch portion is preferably from 150 to 300 mm for the same reasons as described above. Taking into consideration the possibility of using a booster pad in combination, the smallest width is more preferably 180 to 280 mm, even more preferably 200 to 270 mm.

In the present embodiment, the ratio of the distance W1 between the opposite fixed ends of the paired standing gathers to the distance W2 between the opposite elastic members that are arranged most outward in the paired leg gathers, W1/W2, both measured at the smallest width of the crotch portion (see Fig. 2) is 0.67 to 0.81, preferably 0.67 to 0.75. With the smallest width of the diaper 1 in the crotch portion C falling in the range recited for the second aspect of the invention (i.e., 100 to 240 mm), where the ratio W1/W2 is smaller than 0.67, the space between the standing gathers tends to be too small to exhibit the necessary absorbency. With the smallest width of the diaper 1 in the crotch portion C falling in the range recited for the second aspect of the invention (i.e., 100 to 240 mm), where the ratio is greater than 0.81, there would be no pocket left between the standing gathers and the respective leg gathers, which can cause discharged body waste to flood over and leak.

It is preferred for the disposable diaper 1 to have the W1/W2 ratio in the range of 0.67 to 0.81 in an area extending over at least 50 mm in the diaper length direction, particularly in an area extending from the smallest width towards opposite sides by at least 25 mm. The portion having the smallest width is disposed in almost the middle of the diaper length. When the crotch portion has the same width over a prescribed length in the longitudinal direction of the diaper, and that width is the smallest in the width of the crotch portion, the portion which is either in the longitudinal center of the diaper or the nearest to the center preferably is to be the portion having the smallest width.

In the disposable diaper 1, the distance W3 (see Fig. 2) between the fixed end of the standing gathers on each side of the diaper and the elastic member that is arranged most outwardly in the leg gathers on the same side of the diaper is smaller than the width W4 (see Fig. 2) of the standing gathers on each side in at least the area having the smallest width of the crotch portion. The width W4 of the standing gathers is the distance from the fixed end 64 to the free end 63, measured on the plane of the sheet 62.

The width W3 being smaller than the width W4, the diaper 1 easily achieves both the ease of diapering a wearer in a standing position and the absorbing performance. To ensure these properties, a preferred ratio of the width W3 to the width W4, W3/W4, is 0.45 to 0.83.

It is desirable that the relationship between widths W3 and W4 (W3<W4) be satisfied over an area extending at least 50 mm in the diaper length direction, particularly over an area extending from the smallest width towards opposite sides by at least 25 mm.

The way of putting the disposable diaper 1 on a wearer is illustrated in Figs. 4 and 5. As shown in Fig. 4, the rear portion A is applied to the back of a wearer 30, and the opposite waistband portions 10a and 10b are brought around to the front. The waistband portion 10a, which has the band fastening member 11 near the free end, is overlapped on the waistband portion 10b, and the band fastening member 11 of the waistband portion 10a is engaged onto the backsheet side of the waistband portion 10b. The front portion B is then drawn up along the crotch to the front. The front portion fastening members 20 of the front portion B are engaged onto the opposite waistband portions 10a and 10b elastically stretchable parts 14 as shown in Fig. 5. The diaper 1 can also be put on a wearer by first applying the rear portion A to the stomach side of the wearer 30 and fastening the waistband portions 10 on the wearer's back. In this way, the disposable diaper 1 of the present embodiment can easily be put on a wearer.

The disposable diaper of the above-described embodiments produces for example, the following effects (1) to (3).

Effect (1):

The disposable diaper 1 has the low stiffness regions R1 arranged in the region R (the region in the crotch portion C where the absorbent member is disposed) and the low modulus standing gathers 6 set at a high fixing extension ratio. This design renders the diaper 1 extremely easy to put on a wearer in a standing position as well as a lying position.

A conventional fitted disposable diaper is relatively easy to put on a wearer lying on his or her back because one can open the wearer's leg wide. When a wearer is standing, on the other hand, it is difficult to make the wearer open his or her legs wide. The space under the crotch of the standing wearer is of necessity narrow. Therefore, a one must draw a disposable diaper, which is made up of so many members, through the narrow space between the legs. Probably because of this, the wearing position of a disposable diaper that has been put on a wearer in a standing position is mostly below the intended position due to sliding down of the diaper during fastening. In other words, the front waist size of a fastened diaper having been put on a wearer in a standing position is larger than the true front waist size of the wearer. Additionally, the shape of the stomach of a wearer changes between a lying position and a standing position. That is, the front waist size of a wearer is larger in a standing position than in a lying position. The change in diaper's front waist size due to the diaper sliding down as well as the change in the wearer's front waist size due to the change in shape of the wearer's body makes placing a diaper on a standing wearer more difficult.

To solve this problem, it might be considered effective to reduce the lateral stiffness of a disposable diaper. However, a diaper so designed may bunch up badly in its crotch portion and may become leaky. Therefore, such an approach seems to be impractical.

According to the present embodiment, the high extension and low modulus standing gathers stand upright satisfactorily to maintain sufficient space for preventing leakage even when the crotch portion somewhat bunches up. The disposable diaper 1 thus satisfies the demanding performance requirement of leakproofness and ease of diapering. Thus, the disposable diaper 1 not only retains satisfactory leakproofness but provides improved ease in placing a diaper on a wearer in a standing position. When placing a diaper on a wearer in a standing position, one placing the diaper or the wearer himself can easily draw up the front portion through the narrow space between the legs and fasten the front portion at a targeted position in the same way as in placing a diaper on a wearer in a lying position.

The standing gathers of the present invention can be formed by, for example, by adhering at least one elastic member to a gather-forming nonwoven fabric sheet. The elastic member or members have a tensile load increase rate of 1.0 cN/% or less from 20% extension ratio to an effective extension ratio and a tensile load of 20 to 120 cN at the effective extension ratio as a whole. The term "as a whole" as used herein means "as a total of two or more elastic members". The elastic members for standing gathers include elastic threads (e.g., rubber threads, preferably those having a thickness of 450 dtex or smaller), elastic strips (e.g., rubber strips, preferably those having a thickness to width ratio of 0.1 to 1), and elastic films. Materials of the elastic members include natural rubber, synthetic rubbers (e. g., styrene-butadiene, butadiene, isoprene or neoprene rubbers), ethylene-vinyl acetate copolymers, extensible polyolefins, and urethane. In using a plurality of elastic members, it is preferred for each of them to have a tensile load increase rate of 0.5 cN/% or lower from 20% extension ratio to the respective effective extension ratio and a tensile load of 5 to 50 cN at the effective extension ratio.

Effect (2):

Even when the front portion B is detached from the waistband portions and let free from the hand, the front portion B does not droop loosely and maintains the bowllike shape of the portion having faced the wearer's discharge point because of the low stiffness regions R1 in the region R and the low modulus standing gathers 6 fixed at a high extension ratio. This prevents such disadvantages that solid waste may be dropped during diaper change and that part of the diaper may be brought into contact with water or waste in a toilet bowl during excretion.

Effect (3):

The reduced smallest width of the crotch portion, the specific W1/W2 ratio, the relation between W3 and W4 (W3<W4), and the low modulus standing gathers 6 fixed at a high extension ratio make the disposable diaper easy to put on a wearer in a standing position. That is, the disposable diaper can smoothly be put through the narrow space between the legs, and the leg gathers give a snug fit to the wearer's crotch. The low modulus standing gathers fixed at a high extension ratio exhibit excellent standing capabilities to retain the space between the opposite standing gathers. The opposite standing gathers successfully confine discharged body waist therebetween, hardly allowing the waste to flow over. Thus, the disposable diaper exhibits excellent absorbing performance and secures safely against leakage.

The disposable diaper according to the second aspect of the present invention is characterized by being leak-proof from the crotch portion notwithstanding the smaller width of the crotch portion than in conventional disposable diapers. In general, diaper manufacturers may not think of narrowing the crotch portion, where body waist is to be received. In practice, the crotch portion is slightly narrowed only to such an extent as to form an hourglass shape with which to provide a snug fit. The present inventors have found that, in contrast, high leak-proof performance can be secured even with a reduced width of the crotch portion by specifying the W1/W2 ratio, making W3 smaller than W4, and providing the low modulus standing gathers 6 at a high extension ratio. The second aspect of the present invention has thus been established.

Materials of the members making up the disposable diaper 1 according to the above-described embodiment will then be described.

The materials for forming the topsheet 2, the backsheet 3, the standing gather-forming sheet 62, and the like are not limited and selected appropriately from known materials commonly used in conventional disposable diapers.

The absorbent member 4 includes one made of a fiber aggregate and one containing a fiber aggregate and a superabsorbent polymer. The fiber aggregate includes nonwoven fabrics or fiber webs prepared by various processes. In using a superabsorbent polymer in combination, the polymer may be dispersed in the interstices of a fiber aggregate or disposed in the form of a layer between nonwoven fabrics or fiber webs made of fibrous materials. The absorbent member made of a fiber aggregate or a combination of a fiber aggregate and a superabsorbent polymer is preferably wrapped in a soft sheet of paper or liquid-permeable nonwoven fabric.

The elastic members 61 for making standing gathers, the leg elastic members 71, and waist elastic members 51 are known and conventional. Useful forms of these elastic members include threads (e.g., rubber threads), tapes or strips of some width (e.g., rubber strips), and films. Materials of the elastic members include natural rubber, synthetic rubbers (e.g., styrene-butadiene, butadiene, isoprene or neoprene rubbers), ethylene-vinyl acetate copolymers, extensible polyolefins, and urethane.

The fitted disposable diaper of the present invention, including the first and second aspects, is not limited to the above-described embodiment, and various changes and modifications can be made therein without departing from the spirit and scope of the invention.

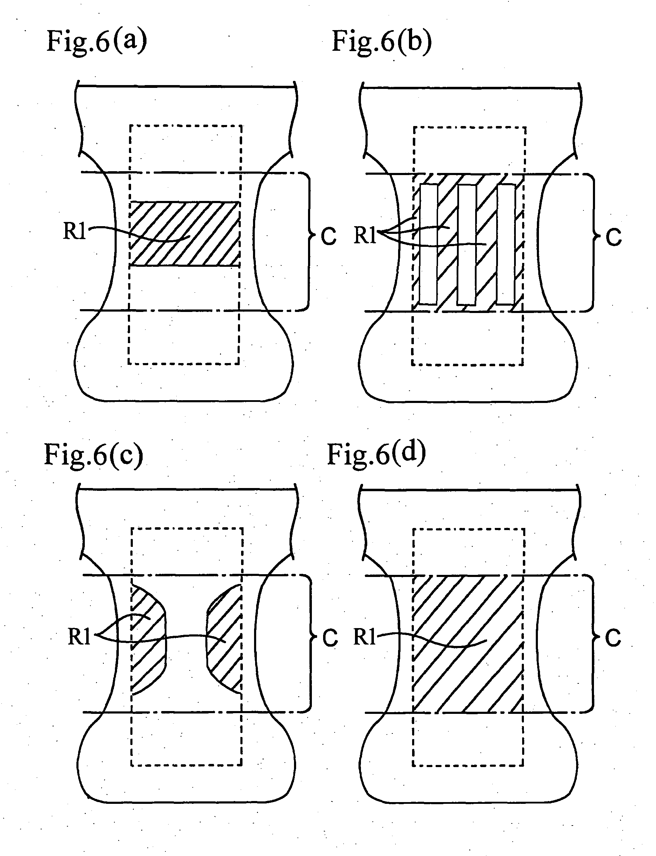

For example, the low stiffness regions R1 according to the first aspect of the invention, which are oppositely provided on both sides of the region R in the diaper length direction in the above-described embodiment, can be arranged as shown in Figs. 6(a) to 6(d). Fig. 6(a) shows a configuration in which a low stiffness region R1 is continuously provided across the middle portion of the region R. Fig. 6(b) depicts a configuration in which a low stiffness region R1 is provided all over the region R except for discretely arranged high stiffness regions. Fig. 6(c) shows another stiffness configuration. Fig. 6(d) displays a configuration in which all the region R is a low stiffness region R1.

The standing gathers 6 of disposable diapers according to the first and second aspects of the invention may have two or more elastic members 61 per side. The leg gathers can be formed by a single elastic member 71 per side. The leg elastic members 71 may be arranged to depict a curve along each arched side edge of the crotch portion. The portions, parts, and the other members constituting the disposable diaper of the present invention are subject to variation in shape or configuration.

The disposable diaper of the present invention is suited for adults, especially for an adult wearer to put on by himself or herself. The disposable diaper of the invention is also fit for babies to toddlers as well.

The following examples further describe and demonstrate embodiments of the present invention. The examples are given solely for the purpose of illustration and are not to be construed as limitations of the present invention.

EXAMPLES 1 TO 4, COMPARATIVE EXAMPLE 1 AND REFERENCE EXAMPLE 1

Disposable diapers having the structure shown in Fig. 1 were produced. In Table 1 are shown the width W12 of the region R, the width W13 and bending stiffness of the high stiffness region R2, the width W11 and bending stiffness of each of the low stiffness regions R1, the fixing extension ratio of the standing gathers, and the tensile characteristics (i.e., effective extension ratio (=fixing extension ratio-30%), tensile load at the effective extension ratio, and tensile load increase rate) of the standing gathers in their non-fixed state.

The standing gathers on each side were formed of two elastic strips adhered to a nonwoven fabric sheet. The absorbent members used in Examples 1 and 2 and Reference Example 1 were fabricated by the aforementioned method (1) so as to have a stiff part in the widthwise middle and a soft part on each longer side thereof. The absorbent members used in Examples 3 and 4 were soft all over, having no stiff part. The absorbent member used in Comparative Example 1 was stiff all over.

Fig. 7 is a graph showing the relationship between extension ratio and tensile stress (load) in standing gathers in their non-fixed state. Curve 1 represents the diapers of Examples 1 to 4, and curve 2 the diapers of Comparative Examples 1 and 2.

In Examples 1 to 4, the W1/W2 ratio was 0.67 to 0.81 over a length of 50 mm or longer in the diaper length direction, and the W3 was smaller than the W4 over a length of 50 mm or longer in the diaper length direction. The W1, W2, W1/W2, W3, and W4 of the diaper obtained in Example 1 are shown in Table 3.

Reference Example 1 is given only for helping the understanding of the effects of the Examples but not for representing a known technique.

TABLE 1

| | W14 (mm) | W12 (mm) | Region 2 | | Region 1 | | Fixing Extension Ratio (%) | Non-fixed State Tensile Characteristics | | |
| | | | W13 (mm) | Bending Stiffness (cN/50mm) | W11 (mm) | Bending Stiffness (cN/50mm) | | Effective Extension Ratio (%) | Tensile Load at Effective Extension Ratio (cN) | Tensile Load Increase Rate (cN/%) |
|---|---|---|---|---|---|---|---|---|---|---|
| Example 1 | 220 | 100 | 70 | 44.5 | 15 | 21.2 | 110 | 80 | 48 | 0.5 |
| Example 2 | 220 | 100 | 60 | 44.5 | 20 | 21.2 | 110 | 80 | 48 | 0.5 |
| Example 3 | 220 | 100 | - | - | 100 | 6.2 | 110 | 80 | 48 | 0.5 |
| Example 4 | 220 | 100 | - | - | 100 | 24.0 | 110 | 80 | 48 | 0.5 |
| Comp. Example 1 | 220 | 100 | 100 | 67.3 | | - | 60 | 30 | 40 | 1.2 |
| Ref. Example 1 | 220 | 100 | 40 | 67.3 | 30 | 10.5 | 60 | 30 | 40 | 1.2 |

Evaluation

The disposable diapers were evaluated for ease of placing a diaper on a wearer in a standing position and leakproofness in accordance with the following test methods. The results obtained are shown in Table 2.

1) Ease of placing a diaper on a wearer in a standing position

The disposable diaper was put on an infant model in a standing position by fastening the waistband portions on the stomach, drawing up the front portion between the legs to bring it to the front, and fastening the front portion to the waistband portions. The ease of placing the diaper was rated "good" or "not good" according to the following standard.

Good: The step of bringing the front portion to the front and the step of pulling up the front portion to the position of the waistband portions and fastening the front portion thereto can be carried out easily.

Not good: Either one or both of the steps of bringing the front portion to the front and the step of pulling up the front portion to the position of the waistband portions and fastening the front portion thereto cannot be carried out easily.

2) Leakproofness in motion

A simulated infant hip model was used for testing. The model is shaped like the hips and buttocks of an infant and is designed to perform a walking movement in its standing position and to discharge artificial urine through a tube from the crotch. The disposable diaper was put on the infant hip model with its front waist end level with the model's navel. The fomodel was operated to make a 5-minute walking movement at a pace of 150 steps per minute. After stopping the walking movement, 80 g of artificial urine was poured through the tube at a rate of 5 g/sec, and the model was again operated to make the same walking movement for an additional 5 minutes. The model was laid on its side, and 40 g of artificial urine was poured at a rate of 5 g/sec. At the point in time when the pouring was completed, whether any leak occurred was inspected with the naked eye. Where no leaks was observed, an additional 40 g portion of the artificial urine was poured. Pouring another 40 g portion of artificial urine was repeated until a leak occurred. The total amount of the artificial urine poured till leakage was taken as a measure of leakproofness in motion.

TABLE 2

|  | Ease of Placing a Diaper | Leakproofness in Motion (g) |
|---|---|---|
| Example 1 | good | 160 |
| Example 2 | good | 160 |
| Example 3 | good | 160 |
| Example 4 | good | 160 |
| Comp. Example 1 | not good | 120 |
| Ref. Example 1 | not good | 120 |

COMPARATIVE EXAMPLE 2 AND REFERENCE EXAMPLE 2

A disposable diaper was produced in the same manner as in Example 1, except for changing the dimensions as shown in Table 3 and using standing gathers having the tensile characteristics indicated by curve 2 of Fig. 7 (Comparative Example 2). A disposable diaper was produced in the same manner as in Example 1, except for changing the dimensions as shown in Table 3 (Reference Example 2).
The disposable diapers of Example 1, Comparative Example 2, and Reference Example 2 were evaluated for its capability of holding soft stool according to the following test method. The results obtained are shown in Table 3.

3) Shape retention for holding loose stool (capability of blocking spread of loose stool)

A simulated infant model was used for testing. The model is shaped like the hips and buttocks of an infant and is designed to change its position from standing to sitting and vice versa, to perform a walking movement in its standing position, to open and close its legs in its sitting position, and to discharge artificial urine and artificial stool through tubes from the crotch. The disposable diaper was put on the infant model with its front waist end level with the model's navel. The model was operated to make a 5-minute walking movement in its standing position at a pace of 150 steps per minute. After stopping the walking movement, 80 g of artificial urine was poured through one of the tubes at a rate of 5 g/sec, and the model was again operated to make the same walking movement for an additional 5 minutes. The model was then made to sit on its buttocks with its legs open, and 160 g of artificial stool was introduced at a rate of 10 g/sec. The model was again operated to repeat the cycle of 5-minute walking in a standing position followed by sitting and opening the legs. The disposable diaper was removed to observe the spread of the artificial stool. Where the artificial stool had spread over the standing gathers, the diaper was rated "not good". Where it had not, the diaper was rated "good".

TABLE 3

| | W1 (mm) | W2 (mm) | W1/W2 | W3 (mm) | W4 (mm) | Shape Retention |
|---|---|---|---|---|---|---|
| Example 1 | 145 | 205 | 0.71 | 30 | 36.5 | good |
| Camp. Example 2 | 100 | 120 | 0.83 | 10 | 36.5 | not good |
| Ref. Example 2 | 150 | 228 | 0.66 | 39 | 43.5 | not good |

The disposable diaper according to embodiments of the present invention, including the first and second aspects, is a flat type with oppositely provided waistband portions that are adapted to be fastened together on the stomach side or the back side of a wearer. The disposable diaper of the present invention is easy to put on whether a wearer is in a lying position or a standing position and exhibits excellent absorbing performance and high leakproofness.
The invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

**Claims**

1.  A disposable diaper of a flat type comprising a rear portion that is to be applied to the back of a wearer, a front portion that is to be applied to the stomach of the wearer, and a crotch portion located between the rear portion and the front portion, the rear portion having a waistband portion oppositely extending from each side thereof, at least one of the waistband portions having a band fastening member near the free end thereof so that the opposite waistbands are fastened together on the front side of the wearer, wherein
    a pair of standing gathers are formed on the longer sides of the diaper,
    a low stiffness region R1 having a bending stiffness of 25 cN/50 mm or lower in the diaper width direction is provided in a region R having the absorbent member in its thickness direction in the crotch portion,
    the standing gathers on each side are fixed at an extension ratio of 100% or higher, and
    the tensile characteristics of the standing gathers on each side measured in their state not fixed to the diaper are such that the tensile load required to extend to an effective extension ratio is 20 to 120 cN and that the increase rate of tensile load required for extending from an extension ratio of 20% up to the effective extension ratio is 1.0 cN/% or lower, the effective extension ratio being 30% lower than the fixing extension ratio.

2.  The disposable diaper according to claim 1, wherein the region R has a high stiffness region R2 having a width of at least 50 mm across the diaper and a bending stiffness of higher than 25 cN/50 mm in the diaper width direction.

3.  The disposable diaper according to claim 1 or 2, wherein the low stiffness region R1 is an oblong region provided along each longer side edge of the absorbent member in the region R.

4.  The disposable diaper according to any one of claims 1 to 3, which is for an infant, wherein the smallest width of the crotch portion is 100 to 240 mm.

5.  The disposable diaper according to any one of claims 1 to 3, which is for an adult, wherein the smallest width of the crotch portion is 150 to 300 mm.

6.  A disposable diaper of a flat type comprising a rear portion that is to be applied to the back of a wearer, a front portion that is to be applied to the stomach of the wearer, and a crotch portion located between the rear portion and the front portion, the rear portion having a waistband portion oppositely extending from each side thereof, at least one of the waistband portions having a band fastening member near the free end thereof so that the opposite waistbands are fastened together on the front side of the wearer, wherein

a pair of standing gathers and a pair of leg gathers are oppositely formed by fixing respective elastic members on longer sides of the diaper,

the smallest width of the crotch portion is 100 to 240 mm,

the ratio of the distance W1 between the opposite fixed ends of the paired standing gathers to the distance W2 between the opposite elastic members that are arranged most outwardly in the paired leg gathers, W1/W2, both measured at the smallest width of the crotch portion is 0.67 to 0.81,

the distance W3 between the fixed end of the standing gathers on each side of the diaper and the elastic member that is arranged most outwardly in the leg gathers on the same side of the diaper as measured at the smallest width of the crotch portion is smaller than the width W4 of the standing gathers on each side as measured at the smallest width of the crotch portion

the standing gathers are fixed at an extension ratio of 100% or higher, and

the tensile characteristics of the standing gathers on each side measured in their state not fixed to the diaper are such that the tensile load required to extend to an effective extension ratio is 20 to 120 cN and that the increase rate of tensile load required for extending from an extension ratio of 20% up to the effective extension ratio is 1.0 cN/% or lower, the effective extension ratio being 30% lower than the fixing extension ratio.

7. The disposable diaper according to claim 6, wherein the ratio W1/W2 is 0.67 to 0.81 over an area extending at least 50 mm in the diaper length direction.

8. The disposable diaper according to claim 6 or 7, wherein the distance W3 is smaller than the distance W4 over an area extending at least 50 mm in the diaper length direction.

## Fig.1

Fig.2

Fig.3

Fig.4

10(10b)   10(10a)   30
          11
                        1

20                      20

B

Fig.5

                        30

20                      20

14                      14

C

Fig.6(a)

R1

C

Fig.6(b)

R1

C

Fig.6(c)

R1

C

Fig.6(d)

R1

C

Fig.7

Extension Ratio vs. Load Curve

EP 1 459 718 A1

## EUROPEAN SEARCH REPORT

Application Number
EP 04 00 6386

**European Patent Office**

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 123 694 A (HUFFMAN GLORIA ET AL) 26 September 2000 (2000-09-26) * the whole document * | 1-8 | A61F13/15 |
| X | WO 00/53140 A (WATANABE HISANORI ; ITO HIDEKAZU (JP); KAO CORP (JP); KOYAMA TAKAO (JP) 14 September 2000 (2000-09-14) * the whole document * | 1-8 | |
| X | WO 01/34083 A (ITOH TAKETO ; KASAI TAKAO (JP); KAO CORP (JP); MAEDA KAZUYUKI (JP)) 17 May 2001 (2001-05-17) * the whole document * | 1-8 | |
| X | WO 01/34084 A (ICHIMATA TOSHIAKI ; KAO CORP (JP); TOYOSHIMA HARUKO (JP)) 17 May 2001 (2001-05-17) * abstract; figures 1-4 * | 1-8 | |
| X | WO 00/03670 A (DUCKER PAUL M ; SNEED SCOTT W (US); DRYPERS CORP (US); KLEMP WALTER V) 27 January 2000 (2000-01-27) * the whole document * | 1-8 | TECHNICAL FIELDS SEARCHED (Int.Cl.7) A61F |
| X | US 5 554 142 A (DREIER KIMBERLY A ET AL) 10 September 1996 (1996-09-10) * the whole document * | 1-8 | |
| X | EP 0 391 476 A (PROCTER & GAMBLE) 10 October 1990 (1990-10-10) * the whole document * | 1-8 | |
| P,X | EP 1 384 460 A (KAO CORP) 28 January 2004 (2004-01-28) * the whole document * | 1-8 | |

-/--

The present search report has been drawn up for all claims

| Place of search | Date of completion of the search | Examiner |
|---|---|---|
| Munich | 28 June 2004 | Settele, U |

**European Patent Office**

**EUROPEAN SEARCH REPORT**

Application Number

EP 04 00 6386

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | US 6 706 030 B1 (WATANABE HISANORI ET AL) 16 March 2004 (2004-03-16) * the whole document * ----- | 1-8 | |
| P,A | EP 1 384 458 A (KAO CORP) 28 January 2004 (2004-01-28) * the whole document * ----- | 1-8 | |
| P,A | EP 1 384 457 A (KAO CORP) 28 January 2004 (2004-01-28) * the whole document * ----- | 1-8 | |
| D,A | EP 0 528 282 A (KIMBERLY CLARK CO) 24 February 1993 (1993-02-24) * the whole document * ----- | 1-8 | |

TECHNICAL FIELDS SEARCHED (Int.Cl.7)

The present search report has been drawn up for all claims

| Place of search | Date of completion of the search | Examiner |
|---|---|---|
| Munich | 28 June 2004 | Settele, U |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 00 6386

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2004

| Patent document cited in search report | | Publication date | Patent family member(s) | | Publication date |
|---|---|---|---|---|---|
| US 6123694 | A | 26-09-2000 | EP | 1011576 A1 | 28-06-2000 |
| | | | WO | 9849988 A1 | 12-11-1998 |
| WO 0053140 | A | 14-09-2000 | JP | 2000254171 A | 19-09-2000 |
| | | | JP | 2001161747 A | 19-06-2001 |
| | | | CN | 1335758 T | 13-02-2002 |
| | | | EP | 1164998 A1 | 02-01-2002 |
| | | | WO | 0053140 A1 | 14-09-2000 |
| | | | US | 6706030 B1 | 16-03-2004 |
| WO 0134083 | A | 17-05-2001 | CN | 1382031 T | 27-11-2002 |
| | | | EP | 1231881 A1 | 21-08-2002 |
| | | | WO | 0134083 A1 | 17-05-2001 |
| | | | JP | 2001198158 A | 24-07-2001 |
| | | | TW | 462888 B | 11-11-2001 |
| WO 0134084 | A | 17-05-2001 | JP | 2001137282 A | 22-05-2001 |
| | | | CN | 1371266 T | 25-09-2002 |
| | | | EP | 1232736 A1 | 21-08-2002 |
| | | | WO | 0134084 A1 | 17-05-2001 |
| WO 0003670 | A | 27-01-2000 | AU | 5211099 A | 07-02-2000 |
| | | | BR | 9912278 A | 17-04-2001 |
| | | | CA | 2338053 A1 | 27-01-2000 |
| | | | EP | 1102570 A1 | 30-05-2001 |
| | | | WO | 0003670 A1 | 27-01-2000 |
| US 5554142 | A | 10-09-1996 | AT | 194483 T | 15-07-2000 |
| | | | AU | 4408396 A | 19-06-1996 |
| | | | BR | 9509924 A | 30-09-1997 |
| | | | CA | 2204893 A1 | 06-06-1996 |
| | | | CN | 1173120 A | 11-02-1998 |
| | | | DE | 69518003 D1 | 17-08-2000 |
| | | | DE | 69518003 T2 | 22-03-2001 |
| | | | EP | 0794752 A1 | 17-09-1997 |
| | | | ES | 2147864 T3 | 01-10-2000 |
| | | | JP | 10509898 T | 29-09-1998 |
| | | | KR | 203650 B1 | 15-06-1999 |
| | | | TR | 960505 A2 | 21-07-1996 |
| | | | WO | 9616623 A1 | 06-06-1996 |
| | | | US | 5653703 A | 05-08-1997 |
| | | | ZA | 9510151 A | 30-05-1996 |
| EP 0391476 | A | 10-10-1990 | US | 5021051 A | 04-06-1991 |
| | | | AT | 106227 T | 15-06-1994 |
| | | | AU | 648018 B2 | 14-04-1994 |

EPO FORM P0459

For more details about this annex : see Official Journal of the European Patent Office, No. 12/82

# EP 1 459 718 A1

## ANNEX TO THE EUROPEAN SEARCH REPORT
## ON EUROPEAN PATENT APPLICATION NO.

EP 04 00 6386

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2004

| Patent document cited in search report | | Publication date | Patent family member(s) | | Publication date |
|---|---|---|---|---|---|
| EP 0391476 | A | | AU 5256690 A | | 11-10-1990 |
| | | | BR 9001617 A | | 07-05-1991 |
| | | | CA 2013182 A1 | | 06-10-1990 |
| | | | CN 1048499 A ,B | | 16-01-1991 |
| | | | DE 69009276 D1 | | 07-07-1994 |
| | | | DE 69009276 T2 | | 08-09-1994 |
| | | | DK 391476 T3 | | 20-06-1994 |
| | | | EP 0391476 A2 | | 10-10-1990 |
| | | | ES 2054213 T3 | | 01-08-1994 |
| | | | FI 97776 B | | 15-11-1996 |
| | | | HK 98196 A | | 14-06-1996 |
| | | | IE 63832 B1 | | 14-06-1995 |
| | | | JP 3000059 A | | 07-01-1991 |
| | | | JP 3027163 B2 | | 27-03-2000 |
| | | | MX 164482 B | | 19-08-1992 |
| | | | PH 27167 A | | 02-04-1993 |
| | | | PT 93653 A ,B | | 20-11-1990 |
| | | | TR 25198 A | | 01-01-1993 |
| EP 1384460 | A | 28-01-2004 | JP 2004057414 A | | 26-02-2004 |
| | | | JP 2004105696 A | | 08-04-2004 |
| | | | JP 2004105697 A | | 08-04-2004 |
| | | | CN 1476817 A | | 25-02-2004 |
| | | | EP 1384460 A2 | | 28-01-2004 |
| US 6706030 | B1 | 16-03-2004 | JP 2000254171 A | | 19-09-2000 |
| | | | JP 2001161747 A | | 19-06-2001 |
| | | | CN 1335758 T | | 13-02-2002 |
| | | | EP 1164998 A1 | | 02-01-2002 |
| | | | WO 0053140 A1 | | 14-09-2000 |
| EP 1384458 | A | 28-01-2004 | JP 2004105698 A | | 08-04-2004 |
| | | | CN 1471901 A | | 04-02-2004 |
| | | | EP 1384458 A2 | | 28-01-2004 |
| EP 1384457 | A | 28-01-2004 | CN 1471900 A | | 04-02-2004 |
| | | | EP 1384457 A2 | | 28-01-2004 |
| EP 0528282 | A | 24-02-1993 | AU 2086592 A | | 11-02-1993 |
| | | | BR 9202958 A | | 30-03-1993 |
| | | | CA 2053106 A1 | | 09-02-1993 |
| | | | EP 0528282 A2 | | 24-02-1993 |
| | | | JP 5184623 A | | 27-07-1993 |
| | | | MX 9204018 A1 | | 30-06-1994 |
| | | | ZA 9205013 A | | 28-04-1993 |

EPO FORM P0459

For more details about this annex : see Official Journal of the European Patent Office, No. 12/82

23